(19) Europäisches Patentamt
European Patent Office
Office européen des brevets

(11) **EP 3 773 407 B1**

(12) **EUROPEAN PATENT SPECIFICATION**

(45) Date of publication and mention
of the grant of the patent:
**27.08.2025 Bulletin 2025/35**

(21) Application number: **19774489.9**

(22) Date of filing: **28.03.2019**

(51) International Patent Classification (IPC):
**A61H 23/00** (2006.01)   **A61H 21/00** (2006.01)
**A61H 23/02** (2006.01)

(52) Cooperative Patent Classification (CPC):
**A61B 5/073; A61B 5/6861; A61H 21/00;**
**A61H 23/0254; A61H 23/0263;** A61H 2201/5005;
A61H 2201/5025; A61H 2201/5035;
A61H 2201/5064; A61H 2201/5071;
A61H 2201/5082; A61H 2201/5084;
A61H 2201/5092; A61H 2201/5097;
A61H 2205/083;                                    (Cont.)

(86) International application number:
**PCT/IB2019/052529**

(87) International publication number:
**WO 2019/186445 (03.10.2019 Gazette 2019/40)**

(54) **GASTROINTESTINAL TREATMENT SYSTEM INCLUDING A VIBRATING CAPSULE, AND METHOD OF USE THEREOF**

GASTROINTESTINALES BEHANDLUNGSSYSTEM MIT EINER VIBRIERENDEN KAPSEL UND VERFAHREN ZU DESSEN VERWENDUNG

SYSTÈME DE TRAITEMENT GASTRO-INTESTINAL COMPRENANT UNE CAPSULE VIBRANTE ET SON PROCÉDÉ D'UTILISATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **30.03.2018 GB 201805325**
                **31.05.2018 GB 201808859**

(43) Date of publication of application:
**17.02.2021 Bulletin 2021/07**

(73) Proprietor: **Vibrant Ltd.**
**2069206 Yokne'am (IL)**

(72) Inventors:
• **BEN-TSUR, Lior**
**4267000 Netanya (IL)**
• **MOLNAR, Shai**
**2016200 Shorashim (IL)**
• **SHABAT, Roni**
**1892500 Kfar Yehezkel (IL)**

(74) Representative: **Harrison IP Limited**
**Mereside, Alderley Park**
**Congleton Road**
**Nether Alderley**
**Macclesfield, Cheshire SK10 4TG (GB)**

(56) References cited:
EP-A1- 2 814 376          EP-B1- 2 814 376
WO-A1-2018/055487          WO-A2-2006/045011
CN-A- 105 434 155          CN-A- 106 377 406
CN-B- 105 380 777          CN-U- 205 286 889
US-A1- 2017 135 897          US-A1- 2017 273 863
US-A1- 2017 296 425

(52) Cooperative Patent Classification (CPC): (Cont.)
  A61H 2230/505

**Description**

FIELD OF THE INVENTION

The present invention relates in general to gastrointestinal treatment systems including one or more vibrating capsules, and more particularly, to gastrointestinal treatment systems in which the one or more capsules vibrate, or are adapted to vibrate, at one or more specific times of day. The present invention further relates in general to a system for mitigating at least one effect of jetlag, and specifically to mitigating jetlag using a gastrointestinal treatment system including one or more vibrating capsules.

Examples of prior art capsules are taught by CN106377406A, CN105434155A, WO2018/055487, EP2814376 and WO2006/045011.

Document CN 106 377 406 A discloses a device according to the preamble of claim 1.

SUMMARY OF THE INVENTION

In accordance with the present invention, there is provided a gastrointestinal treatment system including a gastrointestinal capsule as set forth in claim 1 of the appended claims.

SUMMARY OF THE DISCLOSURE

In this description, force values are expressed in gram-force (gf), a non-SI unit commonly used in certain technical fields. To ensure compliance with SI standards, these values must be converted to Newtons (N) using the formula:

$$F\,(N) = F\,(gf) \times 9.80665 \times 10^{-3}$$

where F (N) is the force in Newtons and F (gf) is the force in gram-force.

In some embodiments, the capsule is adapted to be in the operative state following receipt of an activation input, which transitions the capsule from an inoperative state to an operative state.

In some embodiments, the subject is travelling from an origin location to a destination location, and the system is adapted to mitigate jetlag of the subject.

In some such embodiments, the control mechanism is adapted to activate the vibrating agitation mechanism to operate in the first vibrating mode of operation at at least one predetermined time of day according to a time zone of the origin location.

In some such embodiments, the control mechanism is adapted to activate the vibrating agitation mechanism to operate in the first vibrating mode of operation at at least one predetermined time of day according to a time zone of the destination location.

In some such embodiments, the control mechanism is adapted to activate the vibrating agitation mechanism to operate in the first vibrating mode of operation at a first time of day of the at least one predetermined time of day according to a time zone of the origin location and at a second time of day of the at least one predetermined time of day according to a time zone of the destination location.

In some embodiments, the predetermined time of day is selected according to a circadian cycle of the subject.

In some embodiments, the predetermined time of day is selected according to a gastric pH profile of the subject.

In some embodiments, the capsule includes at least one timing mechanism, and is devoid of sensors for sensing an environment thereof.

In some embodiments, the control mechanism is adapted, in response to the activation input, to wait a predetermined delay time, and following the predetermined delay time, at a time coinciding with the at least one predetermined time of day, to activate the vibrating agitation mechanism to operate in the first vibration mode of operation.

In some embodiments, the capsule further includes at least one sensor adapted to provide the activation input.

In some embodiments, the at least one sensor includes an illumination sensor adapted to provide the activation input upon identification of transition of the capsule from an illuminated environment to a dark environment.

In some embodiments, the at least one sensor includes a pressure sensor adapted to provide the activation input upon identification of pressure applied to the capsule, which pressure is indicative of the capsule moving through a pharynx of the subject.

In some embodiments, the at least one sensor includes a temperature sensor adapted to provide the activation input upon identification of transition of the capsule from an area with ambient temperature to an area with a human body temperature.

In some embodiments, the at least one sensor includes an accelerometer adapted to provide the activation input upon identification of an activation motion carried out by a user of the gastrointestinal capsule.

In some embodiments, the at least one sensor includes a moisture sensor adapted to provide the activation input upon identification of transition of the capsule from a dry environment to a humid environment.

In some embodiments, the capsule further includes a timing mechanism, wherein in response to the activation input, the control mechanism is adapted to activate operation of the timing mechanism to track a time of day so as to identify the at least one predetermined time of day for activation of the vibration agitation mechanism.

In some embodiments, the system further includes a control unit, adapted to provide the activation input to the control mechanism of the gastrointestinal capsule.

In some embodiments, the control unit is adapted to provide the activation input following ingestion of the gastrointestinal capsule by the subject. In other embodiments, the control unit is adapted to provide the activation input prior to ingestion of the gastrointestinal capsule by the subject.

In some embodiments, the control unit is adapted to provide to the control mechanism a current time of day, and the control mechanism is adapted to compute a delay time from the current time of day to the at least one predetermined time of day, and to activate the vibrating agitation mechanism following the delay time.

In some embodiments, the control unit further includes a timing device, and is adapted to provide to the capsule, as the activation input, an input signal indicating a current time of day being the at least one predetermined time of day, and the control mechanism adapted, upon receipt of the input signal, to activate the vibrating agitation mechanism to operate in the first vibrating mode of operation.

In some embodiments, the current time of day is a time of day at an origin location of the subject. In other embodiments, the current time of day is a time of day at a destination location of the subject.

In some embodiments, the activation input includes the at least one predetermined time of day.

In some embodiments, the at least one predetermined time of day includes at least one default predetermined time of day. In some embodiments, the at least one predetermined time of day includes at least one time of day coinciding with at least one predetermined mealtime.

In some embodiments, the at least one predetermined time of day includes at least one time of day coinciding with at least one predetermined mealtime in a time zone of the origin location of the subject.

In some embodiments, the at least one predetermined time of day includes at least one time of day coinciding with at least one predetermined mealtime in a time zone of the destination location of the subject.

In some embodiments, the at least one predetermined mealtime includes at least one default mealtime.

In some embodiments, the at least one default mealtime includes a default breakfast time. In some embodiments, the default breakfast time is between 5am and 10am, between 6am and 10am, between 6am and 9am, between 6am and 8am, between 7am and 10am, between 7am and 9am, and between 7am and 8am.

In some embodiments, the at least one default mealtime includes a default lunchtime. In some embodiments, the default lunchtime is between 12pm and 3pm, between 12pm and 2pm, or between 1pm and 3pm.

In some embodiments, the at least one default mealtime includes a default suppertime. In some embodiments, the default suppertime is between 6pm and 10pm, between 7pm and 10pm, between 8pm and 10pm, between 6pm and 9pm, between 7pm and 9pm, or between 6pm and 8pm.

In some embodiments, at least one of the capsule and the control unit includes an input mechanism for receiving subject-specific input from the subject, and wherein the at least one predetermined mealtime includes at least one subject-specific mealtime of the subject.

In some such embodiments, the subject-specific mealtime of the subject is a subject-specific mealtime in the time zone of the origin location of the subject. In other such embodiments, the subject-specific mealtime of the subject is a subject-specific mealtime in the time zone of the destination location of the subject.

In some embodiments, at least one of the capsule and the control unit includes a location sensor adapted to identify a geographical region in which the capsule is located, and wherein the at least one predetermined time of day includes at least one region-specific time of day of the geographical region in which the capsule is located. In some such embodiments, the at least one region-specific time of day includes at least one region-specific mealtime of the geographical region in which the capsule is located.

In some such embodiments, the geographical region is a geographical region of the origin location of the subject. In other such embodiments, the geographical region is a geographical region of the destination location of the subject.

In some embodiments, the activation input additionally includes a vibration protocol to be used by the vibrating agitation mechanism during the first vibrating mode of operation.

In some embodiments, the control mechanism is adapted to activate the agitation vibration mechanism to operate in the first vibrating mode of operation at the at least one predetermined time of day only if a minimum delay duration has passed between receipt of the activation input and the at least one predetermined time of day.

In some embodiments, the vibrating agitation mechanism includes at least a radial agitation mechanism adapted, in the first vibrating mode of operation, to exert radial forces on the housing, in a radial direction with respect to the longitudinal axis of the housing, thereby to cause the vibrations exerted by the housing. In some embodiments, the radial agitation mechanism includes unbalanced weight attached to a shaft of an electric motor powered by the battery.

In some embodiments, the vibrating agitation mechanism includes at least an axial agitation mechanism adapted, in the first vibrating mode of operation, to exert axial forces on the housing, in an axial direction with respect to the longitudinal axis of the housing, thereby to cause the vibrations exerted by the housing. In some embodiments, the axial agitation mechanism includes an electric motor powered by the battery and an urging mechanism, associated with, and driven by, the electric motor, the urging mechanism adapted to exert the axial forces. In some embodiments, the urging mechanism is adapted to exert the axial forces in opposite directions. In some embodiments, the urging mechanism is adapted to deliver at least a portion of the axial forces in a knocking mode.

In some embodiments, the vibrating agitation mechanism is adapted in the first vibrating mode of operation, to exert radial forces on the housing in a radial direction with respect to the longitudinal axis of the housing and to exert axial forces on the housing in an axial direction with respect to the longitudinal axis of the housing, thereby to cause the vibrations exerted by the housing. In some embodiments, the vibrating agitation mechanism includes a radial agitation mechanism adapted to exert the radial forces and a separate axial agitation mechanism adapted to exert the axial forces. In other embodiments, the vibrating agitation mechanism includes a single agitation mechanism adapted to exert the radial forces and the axial forces.

In some embodiments, the housing includes first and second members, and the vibrating agitation mechanism is includes a mechanism adapted to effect a vibration by moving the first member of the housing in the opposite direction relative to the second member of the housing.

In some embodiments, the vibrating mode of operation including a plurality of cycles, each of the cycles including a vibration duration followed by a repose duration, wherein the housing exerts the vibrations during the vibration duration. In some embodiments, the repose duration is greater than the vibration duration.

In some embodiments, the vibration duration is in the range of 0.1 second to 10 seconds, 1 second to 10 seconds, 1 second to 9 seconds, 2 seconds to 9 seconds, 3 seconds to 9 seconds, 3 seconds to 8 seconds, 3 seconds to 7 seconds, 3 seconds to 6 seconds, 4 seconds to 6 seconds, or 5 seconds to 6 seconds.

In some embodiments, the repose duration is in the range of 1 second to 180 seconds, 3 seconds to 180 seconds, 5 seconds to 180 seconds, 5 seconds to 150 seconds, 5 seconds to 120 seconds, 8 seconds to 100 seconds, 8 seconds to 30 seconds, 10 seconds to 80 seconds, 10 seconds to 70 seconds, 10 seconds to 60 seconds, 10 seconds to 50 seconds, 10 seconds to 40 seconds, 10 seconds to 30 seconds, 10 seconds to 20 seconds, or 15 seconds to 20 seconds.

In some embodiments, a duration of each of the plurality of cycles is in the range of 1.1 seconds to 200 seconds, 5 seconds to 200 seconds, 10 seconds to 200 seconds, 10 seconds to 150 seconds, 10 seconds to 100 seconds, 10 seconds to 80 seconds, 10 seconds to 50 seconds, 10 seconds to 40 seconds, 10 seconds to 30 seconds, 15 seconds to 50 seconds, 15 seconds to 40 seconds, 15 seconds to 30 seconds, or 15 seconds to 25 seconds.

In some embodiments, a cumulative duration of the vibrating mode of operation is in the range of 1 hour to 12 hours, 2 hours to 10 hours, 2 hours to 8 hours, 2 hours to 6 hours, 2 hours to 4 hours, or 2 hours to 3 hours. In some embodiments, the cumulative duration is dependent on properties of the battery.

In some embodiments, the vibrating agitation mechanism is configured such that a net force exerted by the housing on the environment is in the range of 50 grams-force to 600 grams-force.

In some embodiments, the vibrating agitation mechanism is configured to exert the forces on the housing to attain a vibrational frequency within a range of 10Hz to 650Hz, 15Hz to 600Hz, 20Hz to 550Hz, 30Hz to 550Hz, 50Hz to 500Hz, 70Hz to 500Hz, 100Hz to 500Hz, 130Hz to 500Hz, or 150Hz to 500Hz.

In some embodiments, the controlling of the vibrating agitation mechanism is effected so as to effect a mechanical stimulation of the wall of the gastrointestinal tract during the at least one predetermined time of day.

In accordance with an exemplary embodiment, not forming part of the present invention, but helping to understand the invention, there is provided a method of treating the gastrointestinal tract of a subject, the method including:

(a) providing the gastrointestinal capsule as described herein;
(b) ingesting the gastrointestinal capsule; and
(c) controlling the vibrating agitation mechanism such that the first vibrating mode of operation occurs at the at least one predetermined time of day.

In accordance with another embodiment, not forming part of the present invention, but helping to understand the invention, there is provided a method of treating the gastrointestinal tract of a subject, the method including:

(a) providing a gastrointestinal capsule, adapted to transit a gastrointestinal tract of the subject, the capsule having:

(1) a housing arranged along a longitudinal axis;
(2) a vibrating agitation mechanism, powered by the battery, the vibrating agitation mechanism adapted such that, in a first vibrating mode of operation, the housing exerts vibrations on an environment surrounding the capsule;
(3) a power supply disposed within the housing and adapted to power the vibrating agitation mechanism; and
(4) a control mechanism adapted, to activate the vibrating agitation mechanism to operate in the first vibrating mode of operation;

(b) receiving at least one capsule activation input;
(c) ingesting the gastrointestinal capsule; and

(d) responsive to the capsule activation input, controlling the vibrating agitation mechanism such that the first vibrating mode of operation occurs at the at least one predetermined time of day.

**In** accordance with yet another embodiment, not forming part of the present invention, but helping to understand the invention, there is provided a method of treating the gastrointestinal tract of a subject, the method including:

(a) providing a gastrointestinal capsule, adapted to transit a gastrointestinal tract of the subject, the capsule having:

(1) a housing arranged along a longitudinal axis;
(2) a vibrating agitation mechanism, powered by the battery, the vibrating agitation mechanism adapted such that, in a first vibrating mode of operation, the housing exerts vibrations on an environment surrounding the capsule;
(3) a power supply disposed within the housing and adapted to power the vibrating agitation mechanism; and
(4) a control mechanism adapted, to activate the vibrating agitation mechanism to operate in the first vibrating mode of operation;

(b) ingesting the gastrointestinal capsule; and
(c) when the gastrointestinal capsule is in an operative state, controlling the vibrating agitation mechanism such that the first vibrating mode of operation occurs at the at least one predetermined time of day.

In some embodiments, the method is adapted for treating an ailment of the gastrointestinal tract of the subject.
In some embodiments, the subject is travelling from an origin location to a destination location, and the method is adapted for mitigating jetlag of the subject.
In some such embodiments, the first vibrating mode of operation occurs at the at least one predetermined time of day according to the time zone of the origin location.
In other such embodiments, the first vibrating mode of operation occurs at the at least one predetermined time of day according to the time zone of the destination location.
In yet other such embodiments, the first vibrating mode of operation occurs, at a first time of day of the at least one predetermined time of day according to the time zone of the origin location, and at a second time of day of the at least one predetermined time of day according to the time zone of the destination location.
In some embodiments, the predetermined time of day is selected according to a circadian cycle of the subject.
In some embodiments, the predetermined time of day is selected according to a gastric pH profile of the subject.
In some embodiments, providing a gastrointestinal capsule includes providing the gastrointestinal capsule in an inoperative state, the method further including, at the capsule, receiving an activation input transitioning the capsule from the inoperative state to the operative state.
In some embodiments, providing the gastrointestinal capsule includes providing the gastrointestinal capsule including at least one timing mechanism, and devoid of sensors for sensing an environment thereof.
In some embodiments, the controlling includes, responsive to the activation input, waiting a predetermined delay time, and following the predetermined delay time, at a time coinciding with the at least one predetermined time of day, activating the vibrating agitation mechanism to operate in the first vibration mode of operation.
In some embodiments, receiving the at least one activation input includes receiving the at least one activation input from at least one sensor forming part of the gastrointestinal capsule.
In some embodiments, the at least one sensor includes an illumination sensor, and the receiving the at least one activation input includes receiving input indicating transition of the capsule from an illuminated environment to a dark environment.
In some embodiments, the at least one sensor includes a pressure sensor, and the receiving the at least one activation input includes receiving input indicating pressure applied to the capsule, which pressure is indicative of the capsule moving through a pharynx of the subject.
In some embodiments, the at least one sensor includes a temperature sensor, and the receiving the at least one activation input includes receiving input indicating transition of the capsule from an area with ambient temperature to an area with a human body temperature.
In some embodiments, the at least one sensor includes an accelerometer, and the receiving the at least one activation input includes receiving the activation input in response to a detected activation motion carried out with the gastrointestinal capsule.
In some embodiments, the at least one sensor includes a moisture sensor, and the receiving the at least one activation input includes receiving input indicating transition of the capsule from a dry environment to a humid environment.
In some embodiments, the capsule further including a timing mechanism, the method further including, in response to the receiving the activation input, activating operation of the timing mechanism to track a time of day so as to identify the at least one predetermined time of day for activation of the vibration agitation mechanism.
In some embodiments, receiving the activation input includes receiving the activation input from a control unit remote from the gastrointestinal capsule.

In some embodiments, receiving the activation input includes receiving the activation input following the ingesting. In other embodiments, receiving the activation input includes receiving the activation input prior to the ingesting.

In some embodiments, receiving the activation input includes receiving a current time of day, and the controlling the vibration agitation mechanism includes computing a delay time from the current time of day to the at least one predetermined time of day and activating the vibrating agitation mechanism to operate in the first vibration mode of operation following the delay time.

In some embodiments, the activation input indicates that a current time of day is the at least one predetermined time of day, and wherein the controlling the vibration agitation mechanism to operate in the first vibration mode of operation occurs immediately following the receiving the activation input.

In some embodiments, the current time of day is the current time of day in the origin location of the subject. In other embodiments, the current time of day is the current time of day in the destination location of the subject.

In some embodiments, the activation input includes the at least one predetermined time of day.

In some embodiments, the at least one predetermined time of day includes at least one default predetermined time of day. In some embodiments, the at least one predetermined time of day includes at least one time of day coinciding with at least one predetermined mealtime.

In some embodiments, the at least one predetermined mealtime is a predetermined mealtime in the time zone of the origin location of the subject. In other embodiments, the at least one predetermined mealtime is a predetermined mealtime in the time zone of the destination location of the subject.

In some embodiments, the at least one predetermined mealtime includes at least one default mealtime.

In some embodiments, the at least one default mealtime includes a default breakfast time. In some embodiments, the default breakfast time is between 5am and 10am, between 6am and 10am, between 6am and 9am, between 6am and 8am, between 7am and 10am, between 7am and 9am, and between 7am and 8am.

In some embodiments, the at least one default mealtime includes a default lunchtime. In some embodiments, the default lunchtime is between 12pm and 3pm, between 12pm and 2pm, or between 1pm and 3pm.

In some embodiments, the at least one default mealtime includes a default suppertime. In some embodiments, the default suppertime is between 6pm and 10pm, between 7pm and 10pm, between 8pm and 10pm, between 6pm and 9pm, between 7pm and 9pm, or between 6pm and 8pm.

In some embodiments, the method further includes, prior to the controlling, receiving subject-specific input relating to at least one subject-specific mealtime of the subject, and wherein the at least one predetermined mealtime includes the at least one subject-specific mealtime.

In some embodiments, the at least one subject-specific mealtime is a subject-specific mealtime in the time zone of the origin location of the subject. In other embodiments, the at least subject-specific mealtime is a subject-specific mealtime in the time zone of the destination location of the subject.

In some embodiments, the method further includes, prior to the controlling, receiving regional information relating to a geographical region in which the gastrointestinal capsule is located, and wherein the at least one predetermined time of day includes at least one region-specific time of day of the geographical region. In some embodiments, the at least one region-specific time of day comprises a region-specific mealtime of the geographical region.

In some embodiments, the geographical region is a geographical region of the origin location of the subject. In other embodiments, the geographical region is a geographical region of the destination location of the subject.

In some embodiments, receiving the regional information includes receiving an identification of the geographical region. In some embodiments, the identification of the geographical region is received from a location sensor. In some embodiments, receiving the regional information includes receiving the at least one region-specific time of day of the geographical region. In some embodiments, receiving the regional information includes receiving the at least one region-specific mealtime of the geographical region.

In some embodiments, receiving regional information occurs in a control unit remote from the gastrointestinal capsule prior to the gastrointestinal capsule receiving the activation input, and wherein the receiving the activation input includes receiving activation input being on the received regional information.

In some embodiments, receiving regional information is carried out by the control mechanism of the gastrointestinal capsule.

In some embodiments, receiving the activation input additionally includes receiving a vibration protocol to be used by the control mechanism to control operation of the vibrating agitation mechanism.

In some embodiments, the vibrating agitation mechanism includes at least a radial agitation mechanism, and the controlling includes controlling the radial agitation mechanism, in the first vibrating mode of operation, to exert radial forces on the housing, in a radial direction with respect to the longitudinal axis of the housing, thereby to cause the vibrations exerted by the housing.

In some embodiments, the vibrating agitation mechanism includes at least an axial agitation mechanism, and the controlling includes controlling the axial agitation mechanism, in the first vibrating mode of operation, to exert axial forces on the housing, in an axial direction with respect to the longitudinal axis of the housing, thereby to cause the vibrations exerted by the housing.

In some embodiments, the controlling includes controlling the vibrating agitation mechanism, in the first vibrating mode of operation, to exert radial forces on the housing in a radial direction with respect to the longitudinal axis of the housing and to exert axial forces on the housing in an axial direction with respect to the longitudinal axis of the housing, thereby to cause the vibrations exerted by the housing.

In some embodiments, the vibrating agitation mechanism includes a radial agitation mechanism adapted to exert the radial forces and a separate axial agitation mechanism adapted to exert the axial forces.

In some embodiments, the vibrating agitation mechanism includes a single agitation mechanism adapted to exert the radial forces and the axial forces.

In some embodiments, the housing includes first and second members, and wherein the controlling the vibrating agitation mechanism includes effecting a vibration by moving the first member of the housing in the opposite direction relative to the second member of the housing.

In some embodiments, controlling the vibrating agitation mechanism includes controlling the vibrating mode of operation to include a plurality of cycles, each of the cycles including a vibration duration followed by a repose duration, wherein the housing exerts the vibrations during the vibration duration.

In some embodiments, the repose duration is greater than the vibration duration.

In some embodiments, the vibration duration is in the range of 0.1 second to 10 seconds, 1 second to 10 seconds, 1 second to 9 seconds, 2 seconds to 9 seconds, 3 seconds to 9 seconds, 3 seconds to 8 seconds, 3 seconds to 7 seconds, 3 seconds to 6 seconds, 4 seconds to 6 seconds, or 5 seconds to 6 seconds.

In some embodiments, the repose duration is in the range of 1 second to 180 seconds, 3 seconds to 180 seconds, 5 seconds to 180 seconds, 5 seconds to 150 seconds, 5 seconds to 120 seconds, 8 seconds to 100 seconds, 8 seconds to 30 seconds, 10 seconds to 80 seconds, 10 seconds to 70 seconds, 10 seconds to 60 seconds, 10 seconds to 50 seconds, 10 seconds to 40 seconds, 10 seconds to 30 seconds, 10 seconds to 20 seconds, or 15 seconds to 20 seconds.

In some embodiments, a duration of each of the plurality of cycles is in the range of 1.1 seconds to 200 seconds, 5 seconds to 200 seconds, 10 seconds to 200 seconds, 10 seconds to 150 seconds, 10 seconds to 100 seconds, 10 seconds to 80 seconds, 10 seconds to 50 seconds, 10 seconds to 40 seconds, 10 seconds to 30 seconds, 15 seconds to 50 seconds, 15 seconds to 40 seconds, 15 seconds to 30 seconds, or 15 seconds to 25 seconds.

In some embodiments, controlling the vibrating agitation mechanism includes controlling the vibrating agitation mechanism such that a cumulative duration of the vibrating mode of operation is in the range of 1 hour to 12 hours, 2 hours to 10 hours, 2 hours to 8 hours, 2 hours to 6 hours, 2 hours to 4 hours, or 2 hours to 3 hours. In some embodiments, the cumulative duration is dependent on properties of the battery.

In some embodiments, in the first vibration mode of operation, the vibrating agitation mechanism is configured such that a net force exerted by the housing on the environment is in the range of 50 grams-force to 600 grams-force.

In some embodiments, in the first vibration mode of operation the vibrating agitation mechanism is configured to exert the forces on the housing to attain a vibrational frequency within a range of 10Hz to 650Hz, 15Hz to 600Hz, 20Hz to 550Hz, 30Hz to 550Hz, 50Hz to 500Hz, 70Hz to 500Hz, 100Hz to 500Hz, 130Hz to 500Hz, or 150Hz to 500Hz.

In some embodiments, controlling of the vibrating agitation mechanism includes controlling the vibrating agitation mechanism so as to effect a mechanical stimulation of the wall of the gastrointestinal tract during the at least one predetermined time of day.

BRIEF DESCRIPTION OF THE FIGURES

The foregoing discussion will be understood more readily from the following detailed description of the invention, when taken in conjunction with the accompanying Figures (1-5B), in which:

Figure 1 is a schematic block diagram of a gastrointestinal treatment system including a vibrating ingestible capsule according to an embodiment of the present invention;

Figure 2 is a schematic flowchart of a method for treating the gastrointestinal tract, wherein the method itself is not forming part of the present invention, the treatment being based on use of an ingestible vibrating gastrointestinal capsule, for example as shown in Figure 1;

Figure 3 is a schematic illustration of the circadian cycle of a person, including suitable times of day for the implementation of the method of Figure 2;

Figure 4 is a graphic illustration of the gastric pH of a person, indicating suitable times of day for the implementation of the method of Figure 2;

Figures 5A and 5B are graphic representation of results of clinical experiments conducted using an ingestible vibrating gastrointestinal capsule as illustrated in Figure 1 and using a method as illustrated in Figure 2.

## DESCRIPTION OF THE PREFERRED EMBODIMENTS

The principles of the inventive gastrointestinal treatment system and method of treating the gastrointestinal tract using the inventive gastrointestinal treatment system, may be better understood with reference to the drawings and the accompanying description.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

For the purposes of this application, the term "subject" relates to a human.

For the purposes of this application, the term "vibrating ingestible capsule" relates to an ingestible capsule adapted to at least intermittently vibrate, for a cumulative duration of at least one minute, in accordance with a vibration protocol of the capsule.

For the purposes of this application, the term "vibrating agitation mechanism" refers to a any type of mechanism that vibrates or causes elements in its vicinity to vibrate, including a vibration motor or engine.

For the purposes of this application, the term "intermittently activated vibrating agitation mechanism" refers to a vibration engine that vibrates and is operative at certain times, and does not vibrate at other times, the activation times being selected by a control mechanism or other control unit controlling the vibration engine.

For the purposes of this application, the term "vibration protocol" relates to a protocol specifying vibration parameters of an intermittently activated vibrating agitation mechanism of a vibrating ingestible capsule. Typically, the vibration protocol relates to an activation delay for initiating vibration (a duration between activation of the capsule and the first activation of the vibration engine), a vibration rate (number of vibration cycles per hour), a vibration duration and a repose duration for each vibration cycle, a vibration frequency, an amount of force exerted by the vibrations, and the like.

For the purposes of this application, the term "treatment procedure" relates to parameters of a treatment utilizing vibrating ingestible capsules, which are typically defined by a treating physician or medical practitioner. For example, the treatment procedure may include the number of capsules to be taken within a specific time duration (e.g. 3 capsules per week, 2 capsules per day), the frequency at which capsules should be taken, the time of day at which capsules should be taken, whether the capsule should be taken with or without food, and the like.

For the purpose of this application, the term "treatment protocol" relates to all aspects of treatment of a subject with a vibrating ingestible capsule, and includes the treatment procedure as well as the vibration protocol to be used for treating the subject.

For the purpose of this application, the term "activation input" relates to an input received by a control mechanism or control mechanism of a vibrating ingestible capsule, which causes the control mechanism or control mechanism of the capsule to activate itself, so as to be able to process inputs and/or to control additional components of the capsule. The activation input may be received from an element forming part of the capsule, such as a sensor sensing specific conditions in which the capsule should be activated, or from a remote source, such as a remote control mechanism, for example by way of a signal transmitted to the capsule, magnetic field applied to the capsule, specific motion applied to the capsule, or any other type of input provided to the capsule from a remote source.

For the purpose of this application, a vibrating ingestible capsule is said to be in an "inoperative state" when the capsule is in a storage condition, intended to preserve the life of a battery thereof. **In** the inoperative state, components of the capsule which are intended to receive or to provide an activation input, such as specific sensors, transceivers, and/or timing mechanisms may be active at least to a minimal degree. However, in the inoperative state, no vibration takes place, and a control mechanism controlling vibration of the capsule is inactive.

For the purpose of this application, a vibrating ingestible capsule is said to be in an "operative state" when the control mechanism of the capsule is processing inputs and data, and can cause a vibrating agitation mechanism of the capsule to vibrate.

For the purpose of this application, the term "jetlag" relates to any symptom resulting from a change in time zones, including, but not limited to, a shift in a person's sleep schedule, acute constipation resulting from travel, and the like.

For the purpose of this application, the term "mitigating jetlag" relates to appreciably decreasing any jetlag symptom, for example by at least 25%. For example, for a traveler who typically suffers from acute constipation for the first 48 hours of travel, jetlag would be mitigated if the traveler suffered from such acute constipation for, at most, the first 36 hours of travel.

For the purposes of this application, the term "chronic constipation" relates to a spontaneous bowel movement (SBM) frequency of at most 3 SBMs per week,

For the purposes of this application, the term "acute constipation" relates to a subject suffering from a specific event of constipation, without necessarily suffering from chronic constipation. For example, the subject may be constipated for several days following travel, or during travel, for example as part of the subject's jetlag symptoms.

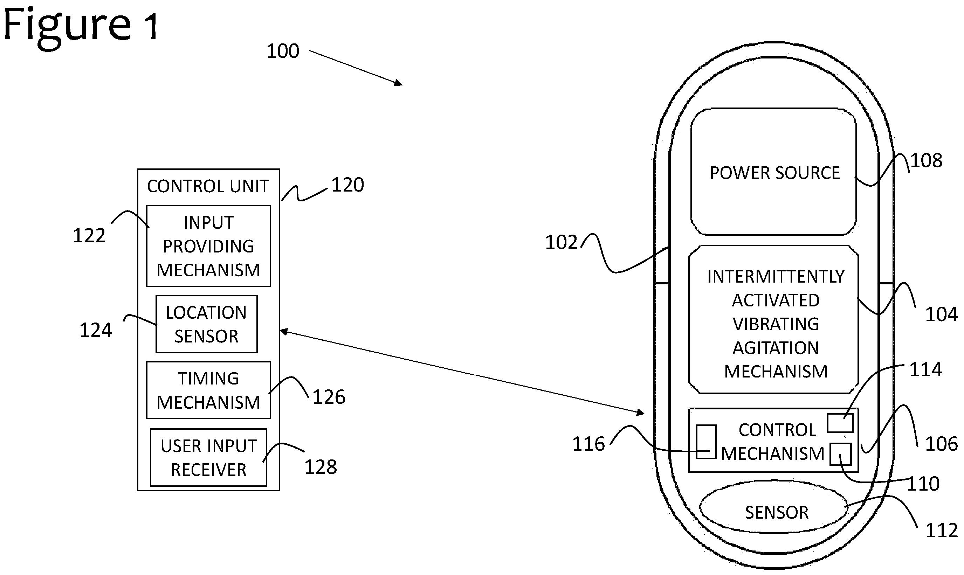

Referring now to the drawings, Figure 1 is a schematic block diagram of a gastrointestinal treatment system **100** including a vibrating ingestible capsule **101** according to an embodiment of the present invention.

As seen in Figure 1, gastrointestinal treatment system **100** includes vibrating ingestible capsule **101**. Capsule **101** includes a capsule housing or shell **102**, arranged along a longitudinal axis and having disposed therein a vibrating agitation mechanism **104**. A control mechanism **106**, which may for example be, or include, a processor, is adapted to control operation of the vibrating agitation mechanism **104**, and at least one power source **108** provides power to vibrating agitation mechanism **104** and control mechanism **106**.

Power source **108** may be any suitable power source, such as one or more alkaline or silver oxide batteries, primary batteries, rechargeable batteries, capacitors and/or supercapacitors.

Intermittently activated vibrating agitation mechanism **104** is adapted to have a vibration mode of operation (also termed the first mode of operation) and a rest mode of operation (also termed the second mode of operation). In the vibration mode of operation, intermittently activated vibrating agitation mechanism **104** is adapted to exert forces on capsule housing **102**, such that capsule housing **102** exerts vibrations on an environment surrounding capsule **101**.

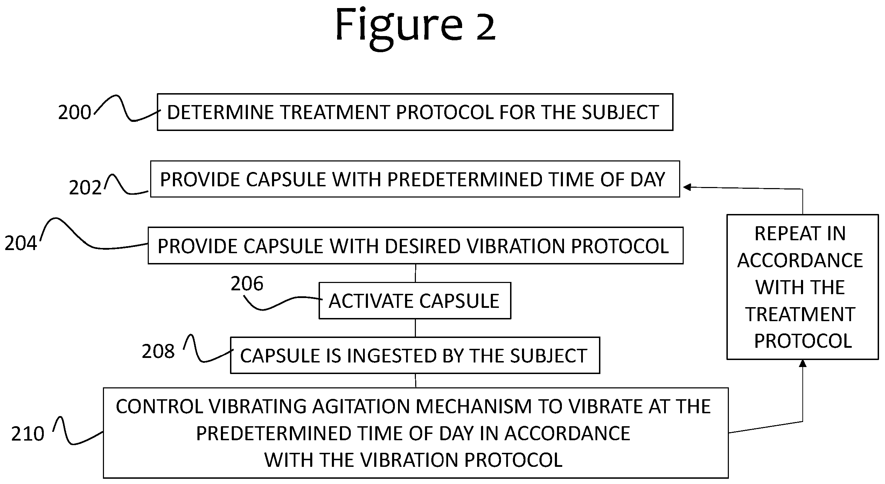

It is a particular feature of the present invention that control mechanism **106** is adapted, in response to receipt of an activation input or when the capsule is in an operative state, to activate vibrating agitation mechanism **104** to operate in the vibrating mode of operation at at least one predetermined time of day, as described in detail hereinbelow with respect to Figure 2.

In some embodiments, the capsule is in an inoperative state, until the receipt of an activation input, which causes control mechanism **106** to transition the capsule from the inoperative state to an operative state.

In some embodiments, control mechanism **106** is functionally associated with, or includes, a timing mechanism **110**, powered by power source **108** and adapted to track at least one time characteristic, such as the time of day, a duration that has passed since an activation input was received, or a duration that has passed since the subject ingested capsule **101**.

In some embodiments, in response to receipt of an activation input, control mechanism **106** is adapted to activate operation of timing mechanism **110** to track a time of day, so as to identify the at least one predetermined time of day for activation of vibration agitation mechanism **104**.

In some embodiments, capsule **101** is devoid of any sensors for sensing an environment thereof. In some such embodiments, control mechanism **106** is adapted, in response to receipt of an activation input, to wait a predetermined delay time, and following the predetermined delay time, at a time coinciding with the at least one predetermined time of day, to activate vibrating agitation mechanism **104** to operate in the first vibration mode of operation.

In other embodiments, such as the embodiment illustrated in Figure 1, capsule **101** further includes at least one sensor **112**, functionally associated with control mechanism **106**. The at least one sensor **112** may be adapted to sense at least one parameter within capsule **101** or in an environment of capsule **101**, and may include a temperature sensor, an illumination sensor, a moisture sensor, a pressure sensor, an accelerometer, or any other suitable sensor. In some embodiments, the at least one sensor **112** is adapted to identify a specific condition in capsule **101** or in the vicinity thereof, and to provide an activation input to control mechanism **106** in response to identification of the condition. For example, in some embodiments the condition is indicative of the subject ingesting capsule **101**.

For example, in some embodiments sensor **112** may include an illumination sensor, adapted to identify transition of capsule **101** from an illuminated environment (e.g. outside the human body) to a dark environment (e.g. within the human body) and to provide an activation input in response to identification of such a transition.

As another example, in some embodiments sensor **112** may include a pressure sensor adapted identify pressure applied to the capsule **101**, which pressure is indicative of the capsule moving through a pharynx of the subject, and to provide an activation input in response to identification of such pressure.

As a further example, in some embodiments sensor **112** may include a temperature sensor adapted to identify transition of capsule **101** from an area with ambient temperature (e.g. outside the human body) to an area with a human body temperature and to provide an activation input in response to identification of such a transition.

As another example, in some embodiments sensor **112** may include a motion or acceleration sensor, such as an accelerometer, adapted to identify an activation motion carried out by a user on capsule **101** and to provide an activation input in response to identification of such a transition. An example of an accelerometer providing an activation input for a gastrointestinal capsule is provided in US Patent Application No. 15/169,065 Filed on May 29, 2016.

As a further example, in some embodiments sensor **112** may include a moisture sensor adapted to identify transition of capsule **101** from a dry area (e.g. outside the human body) to a moist area (e.g. within the human body) and to provide an activation input in response to identification of such a transition.

In some embodiments, such as the embodiment illustrated in Figure 1, capsule **101** further includes a location sensor **114**, such as a GPS or GLONASS receiver, functionally associated with control mechanism **106**. Location sensor **114** may be adapted to identify the geographic location of the capsule.

In some embodiments, system **100** further includes a control unit **120,** which is typically remote from capsule **101,** and which is adapted to provide one or more inputs to the capsule. In some such embodiments, capsule **101** further includes a remote input receiving mechanism **116,** functionally associated with control mechanism **106,** and adapted to receive inputs from an input providing mechanism **122** of control unit **120.**

In some embodiments, control unit **120** may further include a location sensor **124,** such as a GPS or GLONASS receiver, adapted to identify the geographic location of the control unit.

In some embodiments, control unit **120** may further include a timing mechanism **126,** adapted to track at least one time characteristic, such as the time of day, or a duration that has passed since a control instruction was provided to capsule **101.**

In some embodiments, control unit **120** may further include a user input receiver **128,** such as a keyboard, touch screen, or touch pad, adapted to receive input from a user, such as the subject, a medical professional treating the subject, or a caregiver of the subject.

Control unit **120** may be any suitable type of control unit. In some embodiments, control unit may be a suitably configured smart phone or a tablet computer.

In some such embodiments, control unit **120** may provide inputs to capsule **101** by remotely transmitting the inputs from input providing mechanism **122** to remote input receiving mechanism **116,** for example using a short range wireless communication method, such as radio frequency (RF) communication or Bluetooth® communication. One example of such a mechanism for providing input to a capsule is described in US Patent Application 15/132,039 filed April 18, 2016 and entitled "IN VIVO DEVICE AND METHOD OF USING THE SAME".

In some embodiments, control unit **120** is adapted to provide the activation input to control mechanism **106** of capsule **101.** In some such embodiments, control unit **120** provides the activation input prior to the subject ingesting capsule **101,** whereas in other embodiments control unit **120** provides the activation input following ingestion of capsule **101** by the subject.

As discussed hereinabove, control mechanism **106** of capsule **101** is adapted to activate vibrating agitation mechanism **104** to operate in the vibrating mode of operation at at least one specific time of day. That specific time of day may be identified and/or provided to control mechanism **106** by any of a number of methods or mechanism, some exemplary ones of which are described herein.

In some embodiments, control mechanism **106** of capsule **101** is pre-programmed with the predetermined time of day, which may be, for example, a default time of day.

In some embodiments, capsule **101** is adapted to be used to mitigate jetlag of a user travelling from an origin location having an origin time zone to a destination location having a destination time zone. In some such embodiments, the pre-determined time of day is a time of day in the origin time zone. In other such embodiments, the pre-determined time of day is a time of day in the destination time zone. In yet other embodiments, the predetermined time of day is a time of day in a home time zone, which is the time zone at which the subject normally resides, or has been residing for at least a predetermined duration, for example at least one week.

In some such embodiments, timing mechanism **110** provides control mechanism **106** with the current time of day, such that, following receipt of the activation input, or once capsule **101** is in the operative state, control mechanism tracks the current time of day, for example in the origin time zone, the destination time zone, or the home time zone, until arrival of the predetermined time, and then activates vibrating agitation mechanism according to a suitable vibration protocol.

In some embodiments, the predetermined time of day is provided to control mechanism **106** as part of the activation input, or in another input, for example provided by control unit **120.**

In some embodiments, regardless of whether the predetermined time of day is pre-programmed or is provided to control mechanism **106** as an input, the activation input (or another input) provided to control mechanism **106,** for example by control unit **120,** includes the current time of day at the time of providing the activation input, for example in the origin time zone, the destination time zone, or the home time zone. In such embodiments, control mechanism **106** and/or timing mechanism **110** is adapted to compute a delay time from the received current time of day (e.g. the time of the activation input) to the predetermined time of day, to wait the computed delay time and subsequently to activate vibrating agitation mechanism **104** according to a suitable vibration protocol.

In yet another embodiment, not according to the present invention, control mechanism **106** is not provided with the predetermined time of day. Instead, at the predetermined time of day, as indicated by timing mechanism **126,** control unit **120** provides an input to control mechanism **106** indicating that the current time of day is the predetermined time, and that the vibrating agitation mechanism should be activated. Control mechanism **106** is adapted to activate vibrating agitation mechanism **104** to operate in the vibrating mode of operation immediately responsive to receipt of such an input.

In some embodiments, the predetermined time of day is, or coincides with, at least one predetermined mealtime.

In some such embodiments, the predetermined mealtime is a default mealtime. For example, the default mealtime may be a default breakfast time, which may be between 5am and 10am, between 6am and 10am, between 6am and 9am, between 6am and 8am, between 7am and 10am, between 7am and 9am, and between 7am and 8am. As another example, the default mealtime may be a default lunch time, which may be between 12pm and 3pm, between 12pm and 2pm, or between 1pm and 3pm. As yet another example, the default meal time may be a default supper time, which may be between 6pm and 10pm, between 7pm and 10pm, between 8pm and 10pm, between 6pm and 9pm, between 7pm and 9pm, or between 6pm and 8pm.

In some embodiments, the predetermined time of day is a user-specific time of day, or a user-specific mealtime at which the subject typically eats his/her meals. In some such embodiments, user input receiver 128 of control unit 120 (or an input receiver forming part of capsule 101 (not shown)), is adapted to receive, from the user (e.g. the subject or a caregiver of the subject) information about the user-specific time of day or user-specific mealtime. Control unit 120 may then provide the user-specific time of day or the user-specific mealtime to control mechanism 106, for example as part of an activation input or as a separate input. For example, if the subject is used to eating their meals at 11am and 4pm, these times may be provided as input to control unit 120, which may then communicate these predetermined times of day to control mechanism 106 for activation of vibrating agitation mechanism 104 at these times. As another example, if the subject wishes for the capsule to vibrate at 4:00am, the subject may provide this information as input to control unit **120,** which may then communicate this predetermined time to control mechanism **106** for activation of vibrating agitation mechanism **104** at this time.

In some embodiments, the predetermined time of day is suited to the geographic region in which the capsule, the control unit **120,** or the subject, are located. For example, if the subject has ingested the capsule, and has changed time zones since ingesting the capsule (for example flew from New-York to Chicago), the predetermined time of day may be adjusted to correspond to the new time zone in which the user is located. In some embodiments, the predetermined time of day is a region-specific mealtime at which the people in a geographical region at which the subject (or capsule) is located typically eat their meals.

The times at which people typically have supper, or dinner, may vary greatly between different geographical regions. For example, in the U.S. typical supper times are between 5pm and 8pm or between 6pm and 8pm, whereas in Argentina most people only eat their supper between 9pm and 11pm, or even as late as midnight. Thus, the geographical region in which the capsule (and the subject) is located, may be used to identify the typical mealtimes of the subject.

In some such embodiments, location sensor **114** of capsule **101** may identify the location of the capsule, and provide the location or a region of the location to control mechanism **106.** In other embodiments, location sensor **124** of control unit **120** may identify the location of the control unit, and may provide the location or a region of the location to control mechanism **106** as part of the activation input or as part of another input. Control mechanism **106** may then determine the predetermined time of day, or region-specific mealtime, at which the vibrating agitation mechanism **104** should be in the operative mode of operation, based on the region. For example, control mechanism **106** may be preprogrammed with predetermined times of day for specific regions, and may select a suitable pre-programmed predetermined time of day based on the identified region. As another example, control mechanism **106** may access a database (not shown) to find the region-specific time of day, or region-specific mealtimes, for the identified region.

In yet other embodiments, location sensor **124** of control unit **120** may identify the location of the control unit, and may obtain, based on the identified location, the region-specific predetermined time(s) of day or region-specific mealtime(s), for example by accessing a pre-programmed list or by accessing a database (not shown). Control unit **120** may then provide the obtained region-specific predetermined time(s) of day to control mechanism **106,** substantially as described hereinabove.

In some embodiments, in which the capsule **101** is adapted to mitigate jetlag, the predetermined time of day is suited to the geographic region from which the capsule, the control unit **120,** or the subject, originate, or to the origin time zone of the subject. For example, if the subject has ingested the capsule, and has changed time zones since ingesting the capsule (for example flew from New-York to Chicago), the predetermined time of day is preferably maintained at the origin time zone of the origin location of the subject.

In some embodiments, the predetermined time of day is suited to the geographic region at which the capsule, the control unit **120,** or the subject, arrive, or to the destination time zone of the subject. For example, if the subject has ingested the capsule, and has changed time zones since ingesting the capsule (for example flew from New-York to Chicago), the predetermined time of day is preferably maintained at the destination time zone at which the subject has arrived.

In some embodiments, the capsule is adapted to operate in multiple cycles and/or during multiple predetermined times of day. In some such embodiments, in a first cycle or predetermined time of day, the time is suited to the geographic region from which the capsule, the control unit **120,** or the subject, originate, or to the origin time zone of the subject, and in a second cycle or predetermined time of day, the time is suited to the geographic region at which the capsule, the control unit **120,** or the subject, arrive, or to the destination time zone of the subject.

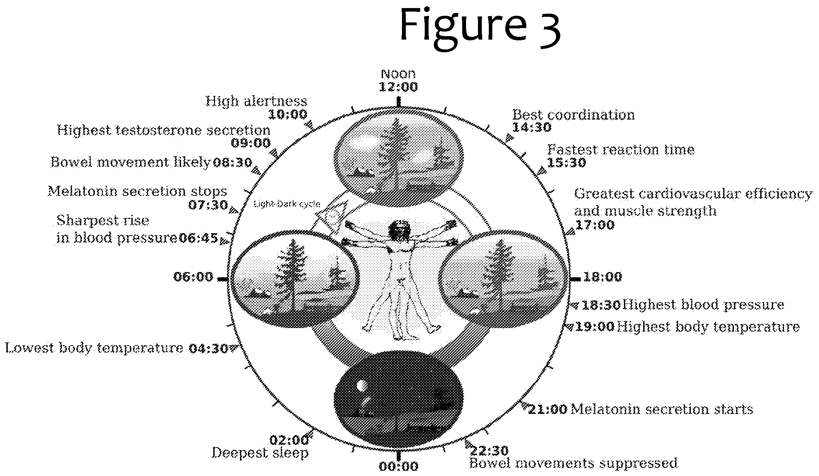

In some embodiments, the predetermined time of day is suited to the circadian cycle of the user, or to a default circadian cycle. For example, the predetermined time of day may be one occurring near a time that, according to the circadian cycle, the user is likely to have a bowel movement, such that operation of the capsule **101** may "assist" the gastrointestinal tract in generating, or completing, such a bowel movement. Additional examples relating to the circadian cycle are described hereinbelow with respect to Figure 3.

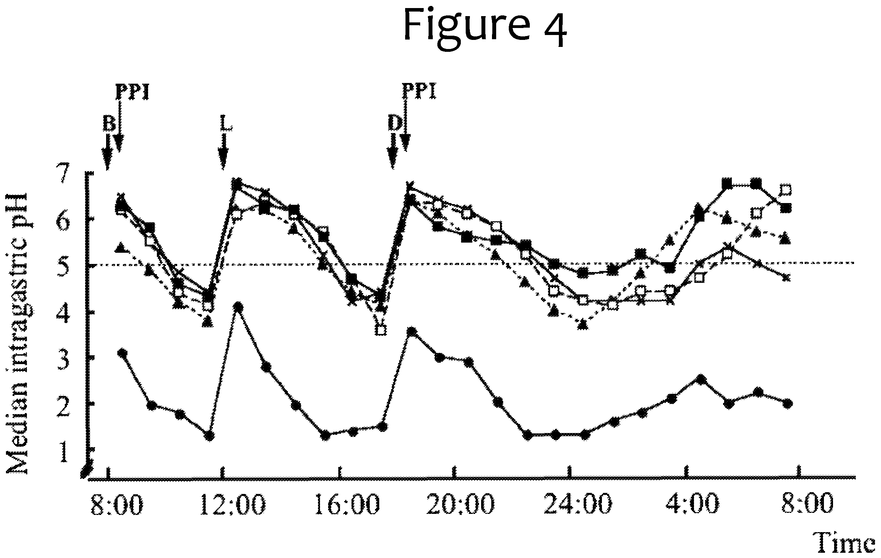

In some embodiments, the predetermined time of day may be selected to be a time of day at which the gastric pH of the user is relatively high, as explained in further detail hereinbelow with respect to Figure 4.

In some embodiments, control mechanism **106** only activates vibrating agitation mechanism in the vibrating mode of operation at the predetermined time, if some minimum threshold duration has passed since capsule **101** was activated to be in the operative state, since receipt of the activation input, or since the user ingested the capsule. For example, the capsule may be pre-programmed such that control mechanism **106** is adapted to activate vibrating agitation mechanism in the vibrating mode of operation from 6am to 8am, and from 6pm to 8pm, and the minimum threshold duration is four hours from ingestion of the capsule. If the subject ingests the capsule at 12pm on Sunday, control mechanism **106** would cause vibrating agitation mechanism **104** to operate in the vibrating mode of operation at 6pm on Sunday, since the predetermined time of 6pm is six hours following ingestion. However, if the subject ingests the capsule at 5pm on Sunday, control mechanism **106** would cause vibrating agitation mechanism **104** to operate in the vibrating mode of operation only at 6am on Monday, since the first predetermined time of 6pm is fewer than four hours following ingestion. Such a threshold duration may be particularly useful when it is desired that the capsule vibrate in a specific region or portion of the gastrointestinal tract, so that the delay time is required for the capsule to reach the specific region.

Relating to the characteristics of vibrating agitation mechanism **104,** the vibrating agitation mechanism may be any suitable mechanism that can be intermittently activated and can apply suitable forces onto capsule housing **102.**

In some embodiments, intermittently activated vibrating agitation mechanism **104** may include a radial agitation mechanism adapted to exert radial forces on capsule housing **102,** in a radial direction with respect to the longitudinal axis of housing **102.** For example, the radial agitation mechanism may include an unbalanced weight attached to a shaft of an electric motor powered by said battery, substantially as described in US Patent Number 9,701,150.

In some embodiments, intermittently activated vibrating agitation mechanism **104** may include an axial agitation mechanism adapted to exert radial forces on the capsule housing **102,** in an axial direction with respect to a longitudinal axis of housing **102.** For example, the axial agitation mechanism may include an electric motor powered by the battery and an urging mechanism, associated with, and driven by, the electric motor, such that the urging mechanism adapted to exert said axial forces, substantially as described in US Patent Number 9,707,150. In some embodiments, the urging mechanism adapted to exert the axial forces in opposite directions. In some embodiments, the urging mechanism is adapted to deliver at least a portion of the axial forces in a knocking mode.

In some embodiments, the forces exerted by intermittently activated vibrating agitation mechanism **104** on capsule housing **102** in the vibration mode of operation include radial forces in a radial direction with respect to the longitudinal axis of the housing and axial forces in an axial direction with respect to the longitudinal axis. In some embodiments, a single agitation mechanism exerts both the radial and the axial forces. In other embodiments, the axial forces are exerted by one agitation mechanism, and the radial forces are exerted by another, separate, agitation mechanism, where both agitation mechanisms form part of intermittently activated vibrating agitation mechanism **104.**

In some embodiments, the intermittently activated vibrating agitation mechanism **104** may include a magnet mounted onto a rotor adapted to exert a magnetic field as well as radial forces on capsule housing **102.** For example, such a magnetic vibration agitation mechanism is described in US Patent Application Number 15/058,216 filed on March 2, 2016 and entitled "PHYSIOTHERAPY DEVICE AND METHOD FOR CONTROLLING THE PHYSIOTHERAPY DEVICE".

In some embodiments, housing **102** may include first and second members, and vibrating agitation mechanism **104** may include a mechanism adapted to effect a vibration by moving the first member of the housing in the opposite direction relative to the second member of the housing, substantially as described in US Patent Number 9,078799.

In the vibrating mode of operation, intermittently activated vibrating agitation mechanism **104** is adapted to have a plurality of vibration cycles, where each cycle includes a vibration duration followed by a repose duration. Forces are exerted by the vibrating agitation mechanism **104** on capsule housing **102** only during the vibration duration, and as such capsule housing **102** only exerts forces on an environment thereof during the vibration duration.

In some embodiments, the number of vibration cycles per hour is in the range of 20 to 400, 40 to 400, 60 to 400, 80 to 400, 40 to 380, 60 to 380, 80 to 380, 40 to 360, 60 to 360, 80 to 360, 100 to 360, 100 to 330, 100 to 300, 100 to 280, 100 to 250, 100 to 220, 100 to 200, 120 to 300, 120 to 280, 120 to 250, 120 to 220, 120 to 200, 150 to 300, 150 to 280, 150 to 250, 150 to 220, 150 to 200, 170 to 300, 170 to 250, 170 to 220, or 170 to 200.

In some embodiments, the repose duration is greater than the vibration duration.

In some embodiments, the vibration duration is in the range of 0.1 second to 10 seconds, 1 second to 10 seconds, 1 second to 9 seconds, 2 seconds to 9 seconds, 3 seconds to 9 seconds, 3 seconds to 8 seconds, 3 seconds to 7 seconds, 3 seconds to 6 seconds, or 4 seconds to 6 seconds.

In some embodiments, the repose duration is in the range of 1 second to 180 seconds, 3 seconds to 180 seconds, 5 seconds to 180 seconds, 5 seconds to 150 seconds, 5 seconds to 120 seconds, 8 seconds to 100 seconds, 8 seconds to 30 seconds, 10 seconds to 80 seconds, 10 seconds to 70 seconds, 10 seconds to 60 seconds, 10 seconds to 50 seconds, 10 seconds to 40 seconds, 10 seconds to 30 seconds, 10 seconds to 20 seconds, or 15 seconds to 20 seconds.

In some embodiments, the total duration of one vibration cycle is in the range of 1.1 seconds to 200 seconds, 5 seconds to 200 seconds, 10 seconds to 200 seconds, 10 seconds to 150 seconds, 10 seconds to 100 seconds, 10 seconds to 80 seconds, 10 seconds to 50 seconds, 10 seconds to 40 seconds, 10 seconds to 30 seconds, 15 seconds to 50 seconds, 15 seconds to 40 seconds, 15 seconds to 30 seconds, or 15 seconds to 25 seconds.

In some embodiments, the cumulative duration of the vibrating mode of operation, or the cumulative duration during which vibration cycles are occurring, is in the range of 1 hour to 12 hours, 2 hours to 10 hours, 2 hours to 8 hours, 2 hours to 6 hours, 2 hours to 4 hours, or 2 hours to 3 hours. It will be appreciated that the cumulative duration of vibration cycles may be dependent on properties of power source **108.**

It will be appreciated by persons skilled in the art that the vibration mode of operation may be intermittent, or interrupted, such that vibrating agitation mechanism **104** is operative in the vibration mode for a first duration, for example 30 minutes, then does have any vibration cycles for a second duration, for example 1 hour, and then is operative in the vibration mode and has vibration cycles for a third duration, for example two hours. The cumulative duration relates to the sum of all durations during which vibrating agitation mechanism **104** was operative in the vibration mode and included vibration cycles, including the vibration duration and the repose duration of the vibration cycle.

In some embodiments, vibrating agitation mechanism **104** is configured to exert forces on the capsule housing **102,** such that a net force exerted by the capsule housing **102** on the environment thereof is in the range of 50 grams force (gf) to 600gf, 50gf to 550gf, 100gf to 550gf, 100gf to 500gf, 150gf to 500gf, 200gf to 500gf, or 200gf to 450gf.

In some embodiments, vibrating agitation mechanism **104** is configured to exert said forces on capsule housing **102** to attain a capsule housing **102** vibrational frequency within a range of 10Hz to 650Hz, 15Hz to 600Hz, 20Hz to 550Hz, 30Hz to 550Hz, 50Hz to 500Hz, 70Hz to 500Hz, 100Hz to 500Hz, 130Hz to 500Hz, or 150Hz to 500Hz.

It will be appreciated that the exact specifications of the capsule, such as the specific frequency and force ranges applicable to a specific capsule, are dependent on the specifications of the power source and of the vibrating agitation mechanism.

It will be further appreciated that a specific capsule may be controlled by the control mechanism such that different vibrational frequencies may be attained and/or different net forces may be exerted, by the capsule in different vibration cycles of the capsule. Due to the natural distinction between subjects, use of multiple different parameters in different vibration cycles of a single capsule would allow the capsule to successfully treat multiple subjects, even if the personal optimal treatment for those subjects is not the same, as there is a higher chance that in at least some of the vibration cycles the activation parameters of the capsule would reach, or be close to, the optimal parameters for each specific subject.

Control mechanism 106 is adapted to control the operation of intermittently activated vibrating agitation mechanism **104.** Such control may include control of any one or more of the force applied by the vibrating agitation mechanism, the vibrational frequency reached, the times in which vibrating agitation mechanism **104** operates in the vibration mode of operation, the vibration duration of each vibration cycle, the repose duration of each vibration cycle, the vibration cycle duration, and cumulative vibration duration of the vibrating agitation mechanisms.

In some embodiments, control mechanism **106** is adapted to receive information relating to the desired vibration protocol from control unit **120,** prior to ingestion of the capsule or to activation thereof, or during the capsule's traversal of the subject's GI tract. For example, the information may be remotely transmitted from control unit **120** to control mechanism **106,** for example using a short range wireless communication method. In some embodiments, the information is transmitted as a list of vibration parameters for effecting the vibration protocol. In some embodiments, the information is transmitted as executable code for effecting the first vibration protocol.

In some embodiments, the information includes one or more of a predetermined time of day, a region in which the control unit is located, a desired number of vibration cycles, a desired vibration duration in each vibration cycle, a desired repose duration in each vibration cycle, a desired cumulative vibration duration, and the like.

In some embodiments, control mechanism **106** is adapted to control vibrating agitation mechanism **104** so that the capsule applies forces to an environment thereof to effect a mechanical stimulation of the wall of the gastrointestinal tract of the subject at the predetermined time(s).

Reference is now additionally made to Figure 2, which is a schematic flowchart of a method for treating the gastrointestinal tract, wherein the method itself is not forming part of the present invention, the treatment being based on use of a gastrointestinal treatment system including a vibrating ingestible capsule, such as capsule **101** of system **100** of Figure 1. For example, the method of treatment illustrated in Figure 2 may be used for treating an ailment of the gastrointestinal tract, or for mitigating jetlag of the user.

It will be appreciated by people of skill in the art that the method described herein may be used for treatment of various ailments of the gastrointestinal tract, including constipation, a sensation of straining while defecating, a sensation of gastric bloating, and gastroparesis.

It will further be appreciated by people of skill in the art that the method described herein may be used for mitigating at least an acute constipation symptom of jetlag of a subject.

As seen at step **200,** initially the treatment protocol for the subject is determined, for example by a treating physician or medical practitioner. The treatment protocol may indicate the number of treatment sessions per week or per other time duration, the time of day at which a capsule should be ingested, a predetermined time of day at which the capsule should be operative, and/or may indicate the vibration protocol of the capsule.

In some embodiments, for example when the treatment is intended to mitigate jetlag of a travelling subject, the treatment protocol may be selected at least partially according to a travel plan of the subject. As such, the treatment protocol may take into consideration, for example, times the subject will be in travelling (e.g. on an airplane, boat, or train), the origin time zone (from which the subject will be leaving), the destination time zone (at which the subject will be arriving), and the time of day at which the subject will be arriving at the destination.

At step **202,** a control mechanism **106** of an ingestible capsule **101** is provided with a predetermined time (or times) of day at which the vibrating agitation mechanism should be operated in the vibrating mode of operation. In some embodiments, the control mechanism may also be provided with a specification of a time zone to which the predetermined time(s) of day should relate, for example an origin time zone or a destination time zone of a travelling subject.

In some embodiments, at step **204** control mechanism **106** may optionally receive, or be programmed with, a desired vibration protocol for the subject.

At step **206,** the capsule may be activated for use, by transitioning the capsule from an inoperative state to an operative state, for example by receipt of an activation input.

The subject ingests the capsule at step **208,** and at step **210** control mechanism **106** control vibrating agitation mechanism **104** such that the vibrating mode of operation occurs at the predetermined time(s) of day.

In some embodiments, providing of the predetermined time(s) at step **202** and/or providing the desired vibration protocol for the subject at step **204** occurs at the time of manufacturing of the capsule, for example by pre-programming the time(s) into the control mechanism.

In some embodiments, providing of the predetermined time(s) at step **202** and/or providing the desired vibration protocol for the subject at step **204** may be effected by a control unit, such as control unit **120** of Figure 1.

For example, control unit **120** may provide to control mechanism **106** the predetermined time of day, a user-specific predetermined time of day, a region-specific predetermined time of day, or a time-zone specific time of day as described hereinabove. In such embodiments, step **202** may be carried out at any time prior to operating to the vibrating agitation mechanism in the vibrating mode of operation at step **210,** and specifically may be carried out prior to ingestion of the capsule by the subject, or following the subject ingesting the capsule.

For example, the programming of the vibration protocol and/or of the predetermined time of day may include remotely transmitting the desired vibration protocol and/or predetermined time of day from the control unit **120** to the control mechanism **106,** for example using a short range wireless communication method. In some embodiments, the desired vibration protocol is transmitted as a list of vibration parameters for effecting the vibration protocol. In some embodiments, the desired vibration protocol is transmitted as executable code for effecting the vibration protocol.

In some embodiments, the control unit provides the predetermined time of day to control mechanism **106** at the predetermined time of day, which is equivalent to giving the command "operate the vibrating agitation mechanism **104** now". In such embodiments, step **202** is carried out following the subject ingesting the capsule at step **208.**

As mentioned above, the capsule is activated for use at step **206.** Typically, the capsule is activated by receipt of an activation input.

As discussed hereinabove, in some embodiments the activation input may be received from the control unit **120** or from sensors within the capsule sensing that the capsule has been ingested or that a user has carried out an activation motion with the capsule.

In some embodiments, the capsule is activated prior to the user ingesting the capsule at step **208,** for example by a signal from the control unit or by the user carrying out an activation motion. In other embodiments, the activation input is provided at the time of ingestion or immediately thereafter, for example by sensors sensing a change in the environment of the capsule due to its ingestion, as described at length hereinabove. In yet other embodiments, the activation input may be provided remotely when the capsule is already in the body of the subject, for example by remote communication from control module **120.**

Following activation of capsule **101,** or together therewith, capsule **101** is ingested by the subject, and begins to travel through the gastrointestinal tract of the subject, as seen at step **208.**

Operation of vibrating agitation mechanism **104** in the vibrating mode of operation at step **210** effects vibration of capsule housing **102,** as described hereinabove, such that the housing exerts vibrations on the environment surrounding the capsule. Specifically, vibration of capsule housing **102** may be intended to effect a mechanical stimulation of the wall of the gastrointestinal tract at the predetermined time of day.

In some embodiments, for example when the method of Figure 2 is used to treat an ailment of the gastrointestinal tract, a treatment session as defined in steps **202 to 210** may be repeatedly administered to the subject as specified in the treatment protocol for the subject, determined or obtained at step **200.** In some embodiments, the treatment protocol includes administering a plurality of treatment sessions to the subject. In some embodiments, the treatment protocol includes administering at least one treatment session to the subject per week, over a treatment period of at least two weeks, at least at least three weeks, at least four weeks, at least five weeks, at least six weeks, or at least eight weeks. In some embodiments, the treatment protocol includes administering 1 to 7 treatment sessions per week, 3 to 14 treatment sessions per two weeks, 2 to 7 treatment sessions per week, 5 to 14 treatment sessions per two weeks, 3 to 7 treatment sessions per week, 7 to 14 treatment sessions per two weeks, 4 to 7 treatment sessions per week, or 5 to 7 treatment sessions per week.

Reference is now made to Figure 3, which is a schematic illustration of the circadian cycle of a person, including suitable times of day for the implementation of the method of Figure 2. The times of day shown in Figure 3, and the corresponding body activities or characteristics at those times of day, are known in the art, and have been shown in medical and scientific research.

As will be noted, according to the typical circadian cycle shown in Figure 3, a bowel movement is likely around 8:30am, and bowel movements are suppressed around 10:30pm (22:30).

As shown in the Examples below, Applicants have discovered that setting a vibrating ingestible capsule, such as the capsule described hereinabove with respect to Figure 1, to vibrate during the morning hours (e.g. around 7:00-8:00am) significantly increases the number of SBMs experienced by users in those hours.

Without wishing to be bound by theory, Applicants surmise that vibration during the morning hours, at which bowel movements are likely according to the circadian cycle, affects the walls of the gastrointestinal tract and promotes peristaltic movement, thereby assisting in completion of the bowel movement the body is likely to be promoting at that time.

As shown in the Examples below, Applicants have further discovered that setting a vibrating ingestible capsule, such as the capsule described hereinabove with respect to Figure 1, to vibrate during the evening hours (e.g. around 7:00-9:00pm) significantly increases the number of SBMs experienced by users in those hours.

Without wishing to be bound by theory, Applicants surmise that vibration during the evening hours, prior to the body suppressing bowel movements according to the circadian cycle, affects the walls of the gastrointestinal tract and promotes peristaltic movement, thereby assisting in creating an additional bowel movement "cleaning out" the GI tract before bowel movements are suppressed.

Reference is now made to Figure 4, which is a graphic illustration of the gastric pH of a person, indicating suitable times of day for the implementation of the method of Figure 2.

As seen in Figure 4, the gastric pH of a typical person oscillates during the day, and is relatively high at typical mealtimes, and then decreases gradually until the next mealtime. Research has shown gastric pH is sensed by mechanoreceptors in the GI tract, and is tied to peristalsis in the GI tract (see for example "Acid sensing in the gastrointestinal tract" to Holtzer, (Am J Physiol Gastrointest Liver Physiol. 2007 Mar; 292(3): G699-G705., https://www.ncbi.nlm.nih.gov/pmc/articles/ PMC4370835/) in the section about Esophago-gastroduodenal motility).

As shown in the Examples below, Applicants have discovered that setting a vibrating ingestible capsule, such as the capsule described hereinabove with respect to Figure 1, to vibrate during typical mealtimes (during the morning hours and during the evening hours) significantly increases the number of SBMs experienced by users in those hours.

Without wishing to be bound by theory, Applicants surmise that vibration at mealtimes, at which there are changes in the gastric pH according to the graph provided in Figure 4, and during which times there is likely to be peristaltic activity in the GI tract, impacts the walls of the gastrointestinal tract and supports the peristaltic movement, thereby assisting in promoting bowel movements at those times.

### EXAMPLES

Reference is now made to the following examples, which, together with the above description, illustrates the invention in a non-limiting fashion.

### EXAMPLE 1

A study which included 130 participating subjects suffering from constipation was conducted. Half of the participating subjects, termed herein "trial subjects", were treated with a vibrating gastrointestinal capsule according to a treatment protocol, in accordance with the present invention, while the other half, termed herein "sham subjects", were treated with a sham capsule, which appeared and behaved identically to the vibrating gastrointestinal capsule prior to ingesting thereof, but did not vibrate within the subject's alimentary tract.

The treatment protocol included treatment cycles including administering one gastrointestinal capsule per day five times per week, repeated for a treatment duration of six weeks. The administered capsules included a non-chargeable battery as the power source, and a coin-type eccentric vibration motor as the vibrating agitation mechanism.

The capsules administered to the "trial subjects" were programmed to have operate in the vibration mode of operation during the morning hours following an activation time delay of at least 8 hours, and when in the vibration mode of operation, to have vibration treatment cycles including a 3 second vibration duration followed by a 16 second repose duration, for a cumulative treatment duration of 1.5 to 3 hours. During the vibration mode of operation, the force applied by the capsule housing on the surrounding environment was in the range of 200gram-force to 500gram-force, and the vibrational frequency was in the range of 120Hz to 280Hz. Different specific forces were applied to the surrounding environment, and corresponding different vibrational frequencies were attained, in different vibration cycles of the administered capsules.

Due to the activation time delay, it is assumed that vibration was affected when the capsules were disposed in a section of the large intestine of the participating subjects.

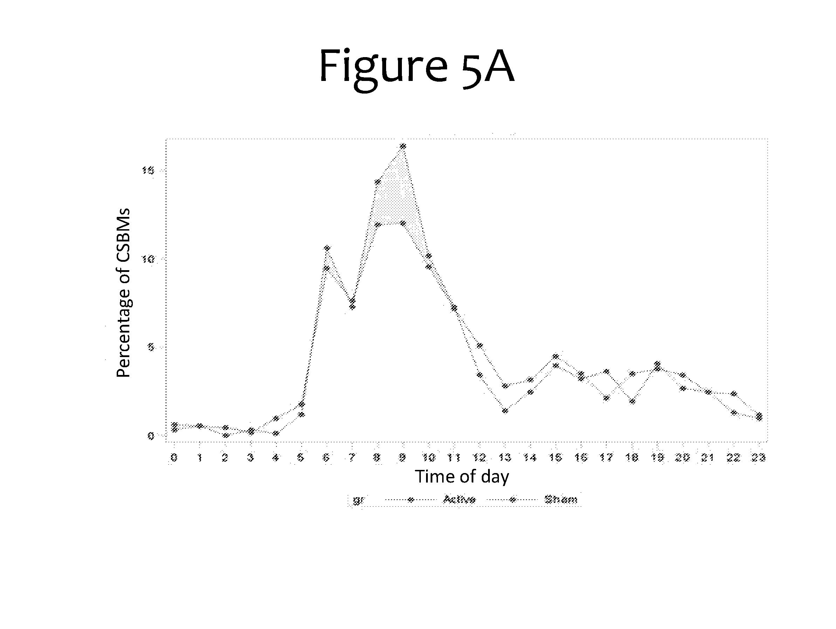

The results of the study are shown in Figure 5A, which illustrates the percentage of complete spontaneous bowel movements (CSBMs) relative to a time from ingestion of the capsules. As seen in Figure 5A, at the times when vibration of the capsules is effected - between 8 and 11 hours after ingestion of the capsules and during the morning hours, the subjects receiving active capsules had a significantly greater number of CSBMs than those receiving sham capsules. As such, the results illustrated in Figure 5A indicate that vibration of the capsules in the morning hours (e.g. at times at which bowel movements are likely according to the circadian cycle), and/or coinciding with mealtimes (e.g. at times at which gastric pH is low), improves the success of the treatment - thereby providing motivation for treating subjects at specific times of day.

### EXAMPLE 2

A study which included 26 participating subjects suffering from constipation was conducted. 16 of the participating subjects, termed herein "trial subjects", were treated with a vibrating gastrointestinal capsule according to a treatment protocol, in two different arms of the study, in accordance with the present invention, while the remaining ten subjects, termed herein "sham subjects", were treated with a sham capsule, which appeared and behaved identically to the vibrating gastrointestinal capsule prior to ingesting thereof, but did not vibrate within the subject's alimentary tract.

The treatment protocol included treatment cycles including administering one gastrointestinal capsule per day five times per week, repeated for a treatment duration of six weeks. The administered capsules included a non-chargeable battery as the power source, and a coin-type eccentric vibration motor as the vibrating agitation mechanism.

The capsules administered to the "trial subjects" were programmed to have operate in the vibration mode of operation during the early morning hours following an activation time delay of at least 8 hours, and to operate in the vibration mode of operation again during the afternoon hours. The capsules were programmed, when in the vibration mode of operation, to have vibration treatment cycles including a 3 second vibration duration followed by a 16 second repose duration, for a cumulative treatment duration of 1.5 to 3 hours. During the vibration mode of operation, the force applied by the capsule housing on the surrounding environment was in the range of 200gram-force to 500gram-force, and the vibrational frequency was in the range of 120Hz to 280Hz. Different specific forces were applied to the surrounding environment, and corresponding different vibrational frequencies were attained, in different vibration cycles of the administered capsules.

Due to the activation time delay, it is assumed that vibration was affected when the capsules were disposed in a section of the large intestine of the participating subjects.

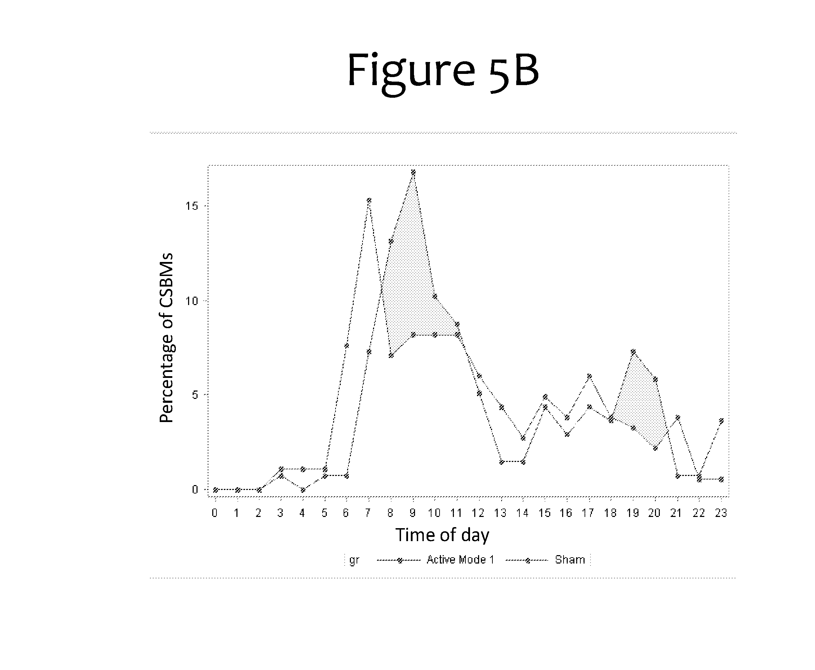

The results of the study are shown in Figure 5B, which illustrates the percentage of complete spontaneous bowel movements (CSBMs) relative to a time from ingestion of the capsules. As seen in Figure 5B, at the times when vibration of the capsules is effected - between 8 and 11 am, and between 7 and 9 pm, the subjects receiving active capsules had a significantly greater number of CSBMs than those receiving sham capsules. As such, the results illustrated in Figure 5B indicate that improvement of the subject's symptoms of the subject's symptoms by addition of CSBMs is coincidental with times at which the capsule vibrates, thus demonstrating the efficacy of the capsules, and the benefit in activating the capsules to operate at specific times of day, which coincide with mealtimes and/or at which the circadian cycle indicates that a bowel movement is likely and prior to bowel movements becoming unlikely according to the circadian cycle.

It will be appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A gastrointestinal treatment system including a gastrointestinal capsule (101) adapted to treat a gastrointestinal tract of a subject following ingestion of said gastrointestinal capsule, for treatment of constipation, said gastrointestinal capsule comprising:

   (a) a housing (102) having a longitudinal axis;
   (b) a vibrating agitation mechanism (104), said vibrating agitation mechanism adapted such that, in a first vibrating mode of operation, said housing exerts vibrations on an environment surrounding said gastrointestinal capsule; and
   (c) a power supply (108) disposed within said housing and adapted to power said vibrating agitation mechanism; **characterised by**
   (d) a programmable control mechanism (106) adapted to be programmed, in response to receipt of an activation input, to compute a delay time from the time of receipt of the activation input to at least one predetermined time of day, to wait the computed delay time, and subsequently to activate said vibrating agitation mechanism to operate in said first vibrating mode of operation at said at least one predetermined time of day , and
   (e) a timing mechanism forming part of, or functionally associated with, the programmable control mechanism, the timing mechanism being adapted to track at least one time characteristic, the at least one time characteristic including at least one of the time of day, a duration that has passed since the activation input was received, and a duration that has passed since the subject ingested said gastrointestinal capsule.

2. The gastrointestinal treatment system of claim 1, wherein said programmable control mechanism (106) is adapted to be programmed such that the time at which said vibrating agitation mechanism operates in said first vibrating mode of operation is at least one predetermined time of day according to at least one of a time zone of an origin location from which the subject is travelling and a time zone of a destination location to which the subject is travelling.

3. The gastrointestinal treatment system of claim 2, wherein said at least one predetermined time of day includes a first predetermined time of day, and a second predetermined time of day, different from the first predetermined time of day, wherein said programmable control mechanism (106) is adapted to be programmed such that a time at which said vibrating agitation mechanism operates in said first vibrating mode of operation is said first predetermined time of day according to a time zone of said origin location and is said second pre-

determined time of day according to a time zone of said destination location.

4. The gastrointestinal treatment system of any one of claims 1 to 3, wherein said capsule is devoid of sensors for sensing an environment thereof.

5. The gastrointestinal treatment system of any one of claims 1 to 4, further comprising a control unit (120), adapted to provide to said programmable control mechanism (106) a current time of day; and said programmable control mechanism is adapted to compute the delay time from said current time of day to said at least one predetermined time of day.

6. The gastrointestinal treatment system of claim 5, wherein said control unit (120) is adapted to provide to said capsule (101), as said current time of day, a time of day at one of an origin location of the subject and a destination location of the subject.

7. The gastrointestinal treatment system of claim 5 or claim 6, wherein at least one of said capsule (101) and said control unit (120) includes a user input receiver (128) for receiving subject-specific input from a subject, and wherein said at least one predetermined time of day comprises at least one subject-specific mealtime of the subject provided as part of the subject-specific input.

8. The gastrointestinal treatment system of claim 5 or claim 6, wherein at least one of said capsule (101) and said control unit (106) includes a location sensor (114) adapted to identify a geographical region in which said capsule is located, and wherein said at least one predetermined time of day comprises at least one region-specific mealtime of said geographical region in which said capsule is located.

9. The gastrointestinal treatment system of any one of claims 1 to 8, wherein said programmable control mechanism (106) is adapted to activate said agitation vibration mechanism (104) to operate in said first vibrating mode of operation at said at least one predetermined time of day only if a minimum delay duration has passed between receipt of said activation input and said at least one predetermined time of day.

10. The gastrointestinal treatment system of any one of claims 1 to 9, wherein said at least one predetermined time of day comprises a default lunchtime.

11. The gastrointestinal treatment system of any one of claims 1 to 10, wherein said at least one predetermined time of day comprises a default suppertime.

**Patentansprüche**

1. Gastrointestinales Behandlungssystem, das eine gastrointestinale Kapsel (101) beinhaltet, die ausgelegt ist, um einen gastrointestinalen Trakt eines Subjekts nach Einnahme der gastrointestinalen Kapsel zur Behandlung von Verstopfung zu behandeln, wobei die gastrointestinale Kapsel Folgendes umfasst:

   (a) ein Gehäuse (102), das eine Längsachse aufweist;
   (b) einen vibrierenden Agitationsmechanismus (104), wobei der vibrierende Agitationsmechanismus ausgelegt ist, sodass in einem ersten vibrierenden Betriebsmodus das Gehäuse Vibrationen auf eine Umgebung ausübt, welche die gastrointestinale Kapsel umgibt; und
   (c) eine Antriebsversorgung (108), die innerhalb des Gehäuses angeordnet und ausgelegt ist, um den vibrierenden Agitationsmechanismus anzutreiben; **gekennzeichnet durch**
   (d) einen programmierbaren Steuermechanismus (106), der ausgelegt ist, um als Reaktion auf Empfang einer Aktivierungseingabe programmiert zu werden, um eine Verzögerungszeit von der Zeit des Empfangs der Aktivierungseingabe zu zumindest einer vorbestimmten Tageszeit zu berechnen, um die berechnete Verzögerungszeit zu warten und um anschließend den vibrierenden Agitationsmechanismus zu aktivieren, um in dem ersten vibrierenden Betriebsmodus zu der zumindest einen vorbestimmten Tageszeit zu arbeiten, und
   (e) einen Zeitplanungsmechanismus, der Teil des programmierbaren Steuermechanismus bildet oder funktionell damit assoziiert ist, wobei der Zeitplanungsmechanismus ausgelegt ist, um zumindest eine Zeiteigenschaft zu verfolgen, wobei die zumindest eine Zeiteigenschaft zumindest eines von der Tageszeit, einer Dauer, die verstrichen ist, seit die Aktivierungseingabe empfangen wurde, und einer Dauer, die verstrichen ist, seit das Subjekt die gastrointestinale Kapsel eingenommen hat, beinhaltet.

2. Gastrointestinales Behandlungssystem nach Anspruch 1, wobei der programmierbare Steuermechanismus (106) ausgelegt ist, um programmiert zu sein, sodass die Zeit, zu welcher der vibrierende Agitationsmechanismus in dem ersten vibrierenden Betriebsmodus arbeitet, zumindest eine vorbestimmte Tageszeit gemäß zumindest einem von einer Zeitzone eines Ursprungsortes, von dem das Subjekt reist, und einer Zeitzone eines Zielortes, zu dem das Subjekt reist, ist.

3. Gastrointestinales Behandlungssystem nach Anspruch 2, wobei die zumindest eine vorbestimmte Tageszeit eine erste vorbestimmte Tageszeit und eine zweite vorbestimmte Tageszeit, die sich von der ersten vorbestimmten Tageszeit unterscheidet, beinhaltet,
wobei der programmierbare Steuermechanismus (106) ausgelegt ist, um programmiert zu sein, sodass eine Zeit, zu welcher der vibrierende Agitationsmechanismus in dem ersten vibrierenden Betriebsmodus arbeitet, die erste vorbestimmte Tageszeit gemäß einer Zeitzone des Ursprungsortes ist und die zweite vorbestimmte Tageszeit gemäß einer Zeitzone des Zielortes ist.

4. Gastrointestinales Behandlungssystem nach einem der Ansprüche 1 bis 3, wobei die Kapsel frei von Sensoren zum Erfassen einer Umgebung davon ist.

5. Gastrointestinales Behandlungssystem nach einem der Ansprüche 1 bis 4, ferner umfassend eine Steuereinheit (120), die ausgelegt ist, um dem programmierbaren Steuermechanismus (106) eine aktuelle Tageszeit bereitzustellen; und
wobei der programmierbare Steuermechanismus ausgelegt ist, um die Verzögerungszeit von der aktuellen Tageszeit zu der zumindest einen vorbestimmten Tageszeit zu berechnen.

6. Gastrointestinales Behandlungssystem nach Anspruch 5, wobei die Steuereinheit (120) ausgelegt ist, um der Kapsel (101) als die aktuelle Tageszeit eine Tageszeit an einem von einem Ursprungsort des Subjekts und einem Zielort des Subjekts bereitzustellen.

7. Gastrointestinales Behandlungssystem nach Anspruch 5 oder Anspruch 6, wobei zumindest eines von der Kapsel (101) und der Steuereinheit (120) einen Benutzereingabeempfänger (128) zum Empfangen von subjektspezifischer Eingabe von einem Subjekt beinhaltet, und wobei die zumindest eine vorbestimmte Tageszeit zumindest eine subjektspezifische Mahlzeit des Subjekts, die als Teil der subjektspezifischen Eingabe bereitgestellt ist, umfasst.

8. Gastrointestinales Behandlungssystem nach Anspruch 5 oder Anspruch 6, wobei zumindest eines von der Kapsel (101) und der Steuereinheit (106) einen Standortsensor (114) beinhaltet, der ausgelegt ist, um eine geografische Region zu identifizieren, in der sich die Kapsel befindet, und wobei die zumindest eine vorbestimmte Tageszeit zumindest eine regionsspezifische Mahlzeit der geografischen Region, in der sich die Kapsel befindet, umfasst.

9. Gastrointestinales Behandlungssystem nach einem der Ansprüche 1 bis 8, wobei der programmierbare Steuermechanismus (106) ausgelegt ist, um den Agitationsvibrationsmechanismus (104) zu aktivieren, um in dem ersten vibrierenden Betriebsmodus zu der zumindest einen vorbestimmten Tageszeit nur zu arbeiten, wenn eine Mindestverzögerungsdauer zwischen Empfang der Aktivierungseingabe und der zumindest einen vorbestimmten Tageszeit verstrichen ist.

10. Gastrointestinales Behandlungssystem nach einem der Ansprüche 1 bis 9, wobei die zumindest eine vorbestimmte Tageszeit eine Standardmittagszeit umfasst.

11. Gastrointestinales Behandlungssystem nach einem der Ansprüche 1 bis 10, wobei die zumindest eine vorbestimmte Tageszeit eine Standardabendessenszeit umfasst.

**Revendications**

1. Système de traitement gastro-intestinal comprenant une capsule gastro-intestinale (101) adaptée pour traiter un tractus gastro-intestinal d'un sujet suite à l'ingestion de ladite capsule gastro-intestinale, pour le traitement de la constipation, ladite capsule gastro-intestinale comprenant :

   (a) un boîtier (102) présentant un axe longitudinal ;
   (b) un mécanisme d'agitation vibrant (104), ledit mécanisme d'agitation vibrant étant adapté de sorte que, dans un premier mode de fonctionnement vibrant, ledit boîtier exerce des vibrations sur un environnement entourant ladite capsule gastro-intestinale ; et
   (c) une alimentation électrique (108) disposée à l'intérieur dudit boîtier et adaptée pour alimenter en électricité ledit mécanisme d'agitation vibrant ; **caractérisé par**
   (d) un mécanisme de commande programmable (106) adapté pour être programmé, en réponse à la réception d'une entrée d'activation, pour calculer un temps de retard entre le moment de la réception de l'entrée d'activation et au moins une heure prédéterminée de la journée, pour attendre le temps de retard calculé, et ensuite pour activer ledit mécanisme d'agitation vibrant pour fonctionner dans ledit premier mode de fonctionnement vibrant à ladite au moins une heure prédéterminée de la journée, et
   (e) un mécanisme de synchronisation faisant partie du mécanisme de commande programmable, ou associé fonctionnellement à celui-ci, le mécanisme de synchronisation étant adapté pour suivre au moins une caractéristique temporelle, l'au moins une caractéristique temporelle comprenant au moins l'une de l'heure de la journée, d'une durée qui s'est écoulée depuis que l'entrée d'activation a été reçue, et d'une durée qui s'est écoulée depuis que le sujet a ingéré ladite capsule gastro-intestinale.

2. Système de traitement gastro-intestinal de la revendication 1, dans lequel ledit mécanisme de commande programmable (106) est adapté pour être programmé de sorte que l'heure à laquelle ledit mécanisme d'agitation vibrant fonctionne dans ledit premier mode de fonctionnement vibrant soit au moins une heure prédéterminée de la journée selon au moins l'un d'un fuseau horaire d'un lieu d'origine en provenance duquel le sujet se déplace et d'un fuseau horaire d'un lieu de destination vers lequel le sujet se déplace.

3. Système de traitement gastro-intestinal de la revendication 2, dans lequel ladite au moins une heure prédéterminée de la journée comprend une première heure prédéterminée de la journée, et une seconde heure prédéterminée de la journée, différente de la première heure prédéterminée de la journée,
   dans lequel ledit mécanisme de commande programmable (106) est adapté pour être programmé de sorte qu'une heure à laquelle ledit mécanisme d'agitation vibrant fonctionne dans ledit premier mode de fonctionnement vibrant est ladite première heure prédéterminée de la journée selon un fuseau horaire dudit emplacement d'origine et est ladite seconde heure prédéterminée de la journée selon un fuseau horaire dudit emplacement de destination.

4. Système de traitement gastro-intestinal de l'une quelconque des revendications 1 à 3, dans lequel ladite capsule est dépourvue de capteurs pour détecter un environnement de celle-ci.

5. Système de traitement gastro-intestinal de l'une quelconque des revendications 1 à 4, comprenant en outre une unité de commande (120), adaptée pour fournir audit mécanisme de commande programmable (106) une heure actuelle de la journée ; et
   ledit mécanisme de commande programmable est adapté pour calculer le temps de retard entre ladite heure actuelle de la journée et ladite au moins une heure prédéterminée de la journée.

6. Système de traitement gastro-intestinal de la revendication 5, dans lequel ladite unité de commande (120) est adaptée pour fournir à ladite capsule (101), en tant que dite heure actuelle de la journée, une heure de la journée à l'un d'un lieu d'origine du sujet et d'un lieu de destination du sujet.

**7.** Système de traitement gastro-intestinal de la revendication 5 ou de la revendication 6, dans lequel au moins l'une de ladite capsule (101) et de ladite unité de commande (120) comprend un récepteur d'entrée utilisateur (128) pour recevoir une entrée spécifique au sujet en provenance d'un sujet, et dans lequel ladite au moins une heure prédéterminée de la journée comprend au moins une heure de repas spécifique au sujet du sujet fournie dans le cadre de l'entrée spécifique au sujet.

**8.** Système de traitement gastro-intestinal de la revendication 5 ou de la revendication 6, dans lequel au moins l'une de ladite capsule (101) et de ladite unité de commande (106) comprend un capteur de localisation (114) adapté pour identifier une région géographique où ladite capsule est située, et dans lequel ladite au moins une heure prédéterminée de la journée comprend au moins une heure de repas spécifique à la région de ladite région géographique où ladite capsule est située.

**9.** Système de traitement gastro-intestinal de l'une quelconque des revendications 1 à 8, dans lequel ledit mécanisme de commande programmable (106) est adapté pour activer ledit mécanisme de vibration d'agitation (104) pour fonctionner dans ledit premier mode de fonctionnement vibrant à ladite au moins une heure prédéterminée de la journée uniquement si une durée de retard minimale s'est écoulée entre la réception de ladite entrée d'activation et ladite au moins une heure prédéterminée de la journée.

**10.** Système de traitement gastro-intestinal de l'une quelconque des revendications 1 à 9, dans lequel ladite au moins une heure prédéterminée de la journée comprend une heure de déjeuner par défaut.

**11.** Système de traitement gastro-intestinal de l'une quelconque des revendications 1 à 10, dans lequel ladite au moins une heure prédéterminée de la journée comprend une heure de souper par défaut.

# Figure 1

100

CONTROL UNIT — 120

122 — INPUT PROVIDING MECHANISM

124 — LOCATION SENSOR

TIMING MECHANISM — 126

USER INPUT RECEIVER — 128

POWER SOURCE — 108

102

INTERMITTENTLY ACTIVATED VIBRATING AGITATION MECHANISM — 104

114

116 — CONTROL MECHANISM — 106

110

SENSOR — 112

EP 3 773 407 B1

# Figure 2

200 — DETERMINE TREATMENT PROTOCOL FOR THE SUBJECT

202 — PROVIDE CAPSULE WITH PREDETERMINED TIME OF DAY

204 — PROVIDE CAPSULE WITH DESIRED VIBRATION PROTOCOL

206 — ACTIVATE CAPSULE

208 — CAPSULE IS INGESTED BY THE SUBJECT

210 — CONTROL VIBRATING AGITATION MECHANISM TO VIBRATE AT THE PREDETERMINED TIME OF DAY IN ACCORDANCE WITH THE VIBRATION PROTOCOL

REPEAT IN ACCORDANCE WITH THE TREATMENT PROTOCOL

Figure 3

Figure 4

# Figure 5A

EP 3 773 407 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 106377406 A
- CN 105434155 A
- WO 2018055487 A
- EP 2814376 A
- WO 2006045011 A
- US 16906516
- US 13203916
- US 9701150 B
- US 9707150 B
- US 05821616
- US 9078799 B

### Non-patent literature cited in the description

- **HOLTZER**. Acid sensing in the gastrointestinal tract. *Am J Physiol Gastrointest Liver Physiol.*, March 2007, vol. 292 (3), G699-G705, https://www.ncbi.nlm.nih.gov/pmc/articles/PMC4370835